# EUROPEAN PATENT APPLICATION

(11) **EP 4 830 739 A1**
(43) Date of publication of application: **28.10.2026**
(21) Application number: 24908192.8
(22) Date of filing: 20.12.2024
(51) Int. Cl.: A61L 27/20

(54) **FILLER COMPOSITION FOR TISSUE REPAIR COMPRISING DOUBLE CROSS-LINKED HYALURONIC ACID AS ACTIVE INGREDIENT USING CROSS-LINKING AGENT HAVING EXCELLENT BIOCOMPATIBILITY**

(30) Priority: 21.12.2023 KR 20230188324
(71) Applicant: Pharma Research Bio Co., Ltd., Gangneung-si, Gangwon-do 25451 (KR)
(72) Inventor: WON, Chee Youb, Seongnam-si Gyeonggi-do 13453 (KR); CHOI, Bok Ryul, Seongnam-si Gyeonggi-do 13453 (KR); EOM, Keun Hee, Seongnam-si Gyeonggi-do 13453 (KR); KIM, Seoyoung, Seongnam-si Gyeonggi-do 13453 (KR); PARK, Sujin, Seongnam-si Gyeonggi-do 13453 (KR)
(74) Representative: Cabinet Beau de Loménie
(86) International application number: PCT/KR2024/020878
(87) International publication number: WO 2025/135910

(57) **Abstract**

The present invention relates to a method for producing crosslinked hyaluronic acid comprising the steps of: (a) crosslinking hyaluronic acid with ornithine, and (b) additionally crosslinking the hyaluronic acid crosslinked with ornithine in step (a) with sodium trimetaphosphate (STMP); a crosslinked hyaluronic acid comprising hyaluronic acid, ornithine and STMP; and a composition for tissue repair, a filler composition, and a composition for *in vivo* administration comprising the crosslinked hyaluronic acid.

## Description

### [Technical Field]

The present invention relates to a method for producing crosslinked hyaluronic acid comprising step (a) of crosslinking hyaluronic acid with ornithine, and step (b) of crosslinking the hyaluronic acid crosslinked with ornithine in step (a) with sodium trimetaphosphate (STMP); crosslinked hyaluronic acid produced by the above method; hyaluronic acid crosslinked with ornithine and STMP; a composition for tissue repair, a filler composition, and a composition for *in vivo* administration, comprising the crosslinked hyaluronic acid; a composition for producing crosslinked hyaluronic acid, a tissue repair biomaterial, a filler, and an *in vivo* administration agent, comprising ornithine, STMP, and hyaluronic acid; a composite composition comprising one or more monomeric units of Chemical Formulas 3 to 7; and a composition for tissue repair, a filler composition, and a composition for *in vivo* administration comprising the same.

### [Background Art]

Biomaterials for tissue repair are used to restore tissue volume in a form suitable for tissue injection, and particularly, the use of fillers for skin wrinkles and other changes in facial lines is also increasing. For example, due to increasing global demand, the global dermal filler market is projected to reach approximately $31 billion in 2031 from about $5 billion in 2021, and the market is expected to record an average annual growth rate of 8% from 2022 to 2031. Among them, hyaluronic acid fillers, due to their high stability and low side effects, serve as a major component of biodegradable fillers for approximately 90% of the total market.

### [Disclosure]

### [Technical Problem]

Generally, when manufacturing hyaluronic acid fillers, crosslinking agents are used to ensure that hyaluronic acid remains in the body without degrading for a long time (KR 10-2051467 B1). However, divinyl sulfone (DVS) and 1,4-butanediol diglycidyl ether (BDDE), which are widely used as hyaluronic acid crosslinking agents, have biological toxicity (Korea Chemical Safety Agency Material Safety Data Sheet System), and thus the development of fillers with excellent safety is minimal.

### [Technical Solution]

One object of the present invention is to provide a method for producing crosslinked hyaluronic acid, comprising: step (a) of crosslinking hyaluronic acid with ornithine; and step (b) of further crosslinking the hyaluronic acid crosslinked with ornithine in step (a) with sodium trimetaphosphate (STMP).

Another object of the present disclosure is to provide crosslinked hyaluronic acid produced by the method of the present invention.

Still another object of the present disclosure is to provide hyaluronic acid crosslinked with ornithine and STMP.

Still another object of the present invention is to provide a composition for tissue repair, a filler composition, and a composition for *in vivo* administration, comprising the crosslinked hyaluronic acid of the present invention.

Still another object of the present invention is to provide a composition for producing crosslinked hyaluronic acid, a composition for producing biomaterial for tissue repair, and a composition for producing a filler, comprising ornithine, STMP, and hyaluronic acid.

Still another object of the present invention is to provide a composite composition comprising one or more monomeric units of Chemical Formulas 3 to 7.

### [Advantageous Effects]

The present invention, which provides excellent biocompatibility and physical properties, can be stably used as a composition for *in vivo* administration, a composition for tissue repair, and a filler composition.

### [Brief Description of the Drawings]

FIG. 1 is a figure showing the biocompatibility evaluation results of various crosslinking agents.
FIG. 2 is a figure showing the storage modulus results according to ornithine concentration.
FIG. 3 is a figure showing the shape of a formulation crosslinked with STMP alone.
FIG. 4 shows the results of confirming the effect of the crosslinking order of ornithine and STMP on storage modulus.
FIG. 5 is a figure showing the effect of ornithine concentration on storage modulus in ornithine and STMP double-crosslinking.
FIG. 6 is a figure showing the effect of STMP concentration on storage modulus in ornithine and STMP double-crosslinking.
FIG. 7 is a figure showing the effect of the molecular weight of hyaluronic acid on storage modulus in ornithine and STMP double-crosslinking.

### [Detailed Description of Preferred Embodiments]

Hereinafter, the present invention will be described in detail as follows. Meanwhile, the description and embodiment of an aspect described herein may be applied to the descriptions and embodiments of other aspects with respect to common matters. In addition, all combinations of various elements described herein fall within the scope of the present invention. Moreover, the literature described in the present invention may be incorporated herein by reference. Further, the scope of the present invention is not limited by the specific description described below.

One aspect of the present invention provides a method for producing crosslinked hyaluronic acid, comprising: step (a) of crosslinking hyaluronic acid with ornithine; and step (b) of crosslinking the hyaluronic acid crosslinked with ornithine in step (a) with sodium trimetaphosphate (STMP).

Step (a) above may comprise a step of mixing hyaluronic acid and ornithine, and step (b) above may comprise a step of further mixing hyaluronic acid crosslinked with ornithine with STMP.

In one embodiment, the method may comprise, prior to step (a), one or more of: a step of dissolving hyaluronic acid and ornithine in a buffer; and a step of adding a coupling agent, but is not limited thereto.

In one embodiment, the method may further comprise, between step (a) and step (b), one or more of: a step of adding a basic solution to cleave unwanted ester crosslinks between hyaluronic acids; a paste step; and a step of adding STMP, but is not limited thereto.

In one embodiment, the method may further comprise, after step (b), one or more of: a step of neutralization by adding an acidic solution; a dialysis step; a grinding step; a final concentration adjustment step; a mixing step; and a sterilization step, but is not limited thereto.

In one embodiment of the foregoing embodiments, the coupling agent may be an activator and/or coupling reagent, and the activator may comprise one or more selected from the group consisting of N-hydroxysuccinimide (NHS), 1-hydroxybenzotriazole (HOBt), 3,4-dihydro-3-hydroxy-4-oxo-1,2,3-benzotriazine (HOOBt), 1-hydroxy-7-azabenzotriazole (HOAt), sulfo-N-hydroxysulfosuccinimide (Sul-NHS), O-(1H-benzotriazole-1-yl)- N,N,N',N'-tetramethyluronium tetrafluoroborate (TBTU), and 4-methylmorpholine (NMM), but is not limited thereto.

In one embodiment of the foregoing embodiments, the coupling reagent may comprise one or more selected from the group consisting of 1-ethyl-3-(3-dimethylaminopropyl)carbodiimide (EDC), 1-cyclohexyl-3-(2-morpholinoethyl) carbodiimide metho-p-toluenesulfonate (CMC), dicyclohexylcarbodiimide (DCC), diisopropylcarbodiimide (DIC), and 2-chloro-dimethoxy-1,3,5-triazine (CDMT).

The coupling agent may comprise two or more coupling agent, and they may be added simultaneously, in sequence, or in reverse order. In one example, in the method, EDC may be added after NHS, but the method is not limited thereto.

Meanwhile, the order of the above steps may be changed.

In addition, the above steps may be performed one or more times, and the number of repetitions may be adjusted by those skilled in the art depending on the desired size or shape of the crosslinked hyaluronic acid.

In one embodiment, in the present invention, the time of crosslinking in step (a) and/or step (b) may be 30 minutes or more. Specifically, it may be 30 minutes to 36 hours, 30 minutes to 24 hours, 30 minutes to 12 hours, 30 minutes to 10 hours, 30 minutes to 8 hours, 30 minutes to 7.5 hours, 30 minutes to 7 hours, 30 minutes to 6.5 hours, 30 minutes to 6 hours, 30 minutes to 5.5 hours, 30 minutes to 5 hours, 30 minutes to 4 hours, 30 minutes to 3 hours, 30 minutes to 2 hours, 1 to 36 hours, 1 to 24 hours, 1 to 12 hours, 1 to 10 hours, 1 to 8 hours, 1 to 7.5 hours, 1 to 7 hours, 1 to 6.5 hours, 1 to 6 hours, 1 to 5.5 hours, 1 to 5 hours, 1 to 4 hours, 1 to 3 hours, 1 to 2 hours, 3 to 48 hours, 3 to 36 hours, 3 to 24 hours, 3 to 12 hours, 3 to 10 hours, 3 to 8 hours, 3 to 7.5 hours, 3 to 7 hours, 3 to 6.5 hours, 3 to 6 hours, 3 to 5.5 hours, 3 to 5 hours, 3 to 4 hours, 6.5 to 48 hours, 6.5 to 36 hours, 6.5 to 24 hours, 6.5 to 12 hours, 6.5 to 10 hours, 6.5 to 8 hours, 6.5 to 7.5 hours, 6.5 to 7 hours, 8 to 48 hours, 8 to 36 hours, 8 to 24 hours, 8 to 12 hours, 8 to 10 hours, 10 to 48 hours, 10 to 36 hours, 10 to 24 hours, 10 to 12 hours, 12 to 48 hours, 12 to 36 hours, 12 to 24 hours, 24 to 48 hours, or 24 to 36 hours, but is not limited thereto.

Other conditions (for example, reaction temperature) of the above steps may include a range of conditions appropriately controlled by those skilled in the art. In one example, the sterilization step may be performed at 100°C to 200°C, 100°C to 150°C, or 121°C, but is not limited thereto.

The present invention is characterized by the development of crosslinked hyaluronic acid with excellent safety, biocompatibility, elasticity, and the like by double-crosslinking hyaluronic acid using both ornithine and STMP, rather than ornithine or STMP alone, and by the fact that it can be used for tissue repair, fillers, *etc*.

Another aspect of the present disclosure provides crosslinked hyaluronic acid produced by the method of the present invention. Specifically, the aspect provides crosslinked hyaluronic acid, comprising hyaluronic acid, ornithine, and STMP.
The method is as described in the aspect above.

As used herein, the term "crosslinked hyaluronic acid" refers to two or more molecules of hyaluronic acid that are crosslinked, and may be used interchangeably with "crosslinked hyaluronic acid".

The crosslinked hyaluronic acid of the present invention may be double-crosslinked with ornithine and STMP, and may be a hydrogel.

As used herein, the term "ornithine" refers to one of the amino acids present in the body and may include L-ornithine and D-ornithine. The ornithine may include physiologically acceptable salts, specifically those in the form of inorganic salts such as hydrochloride and phosphate, or organic salts such as citrate, malate, α-ketoglutaric acid, and aspartate. In one example, it may have the structure of Chemical Formula 1 below, but is not limited thereto. In addition, the ornithine may be obtained by a chemical synthesis method, a fermentation production method, *etc*., or may be a commercially available product.

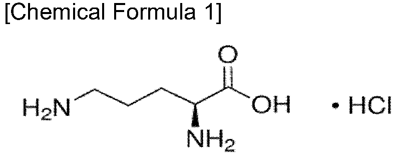

The sodium trimetaphosphate (STMP) of the present invention may have the structure of Chemical Formula 2 below, but is not limited thereto.

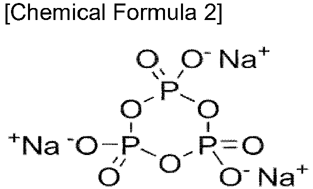

In one embodiment, the hyaluronic acid of the present invention may have an average molecular weight of 10 kDa to 10,000 kDa, specifically 10 kDa to 5,000 kDa, 10 kDa to 4,500 kDa, 10 kDa to 4,000 kDa, 10 kDa to 3,500 kDa, 10 kDa to 3,000 kDa, 10 kDa to 2,500 kDa, 10 kDa to 2,000 kDa, 10 kDa to 1,500 kDa, 10 kDa to 1,000 kDa, 10 kDa to 900 kDa, 10 kDa to 800 kDa, 10 kDa to 700 kDa, 10 kDa to 600 kDa, 10 kDa to 500 kDa, 10 kDa to 450 kDa, 10 kDa to 400 kDa, 10 kDa to 350 kDa, 10 kDa to 300 kDa, 10 kDa to 250 kDa, 10 kDa to 200 kDa, 10 kDa to 180 kDa, 10 kDa to 150 kDa, 10 kDa to 100 kDa, 10 kDa to 50 kDa, 100 kDa to 5,000 kDa, 100 kDa to 4,500 kDa, 100 kDa to 4,000 kDa, 100 kDa to 3,500 kDa, 100 kDa to 3,000 kDa, 100 kDa to 2,500 kDa, 100 kDa to 2,000 kDa, 100 kDa to 1,500 kDa, 100 kDa to 1,000 kDa, 100 kDa to 900 kDa, 100 kDa to 800 kDa, 100 kDa to 700 kDa, 100 kDa to 600 kDa, 100 kDa to 500 kDa, 100 kDa to 450 kDa, 100 kDa to 400 kDa, 100 kDa to 350 kDa, 100 kDa to 300 kDa, 100 kDa to 250 kDa, 100 kDa to 200 kDa, 100 kDa to 180 kDa, 100 kDa to 170 kDa, 100 kDa to 160 kDa, 100 kDa to 150 kDa, 200 kDa to 10,000 kDa, 200 kDa to 5,000 kDa, 200 kDa to 4,500 kDa, 200 kDa to 4,000 kDa, 200 kDa to 3,500 kDa, 200 kDa to 3,000 kDa, 200 kDa to 2,500 kDa, 200 kDa to 2,000 kDa, 200 kDa to 1,500 kDa, 200 kDa to 1,000 kDa, 200 kDa to 900 kDa, 200 kDa to 800 kDa, 200 kDa to 700 kDa, 200 kDa to 600 kDa, 400 kDa to 10,000 kDa, 400 kDa to 5,000 kDa, 400 kDa to 4,500 kDa, 400 kDa to 4,000 kDa, 400 kDa to 3,500 kDa, 400 kDa to 3,000 kDa, 400 kDa to 2,500 kDa, 400 kDa to 2,000 kDa, 400 kDa to 1,500 kDa, 400 kDa to 1,000 kDa, 600 kDa to 10,000 kDa, 600 kDa to 5,000 kDa, 600 kDa to 4,500 kDa, 600 kDa to 4,000 kDa, 600 kDa to 3,500 kDa, 600 kDa to 3,000 kDa, 600 kDa to 2,500 kDa, 600 kDa to 2,000 kDa, 600 kDa to 1,500 kDa, 600 kDa to 1,000 kDa, 800 kDa to 10,000 kDa, 800 kDa to 5,000 kDa, 800 kDa to 4,500 kDa, 800 kDa to 4,000 kDa, 800 kDa to 3,500 kDa, 800 kDa to 3,000 kDa, 800 kDa to 2,500 kDa, 800 kDa to 2,000 kDa, 800 kDa to 1,500 kDa,1,000 kDa to 10,000 kDa, 1,000 kDa to 5,000 kDa, 1,000 kDa to 4,500 kDa, 1,000 kDa to 4,000 kDa, 1,000 kDa to 3,500 kDa, 1,000 kDa to 3,000 kDa, 1,000 kDa to 2,500 kDa, 1,000 kDa to 2,000 kDa, 1,000 kDa to 1,500 kDa, 1,500 kDa to 10,000 kDa, 1,500 kDa to 5,000 kDa, 1,500 kDa to 4,500 kDa, 1,500 kDa to 4,000 kDa, 1,500 kDa to 3,500 kDa, 1,500 kDa to 3,000 kDa, 1,500 kDa to 2,500 kDa, or 1,500 kDa to 2,000 kDa, but is not limited thereto. In addition, the hyaluronic acid of the present invention may be classified into ultra-low molecular weight hyaluronic acid having 10 kDa to 200 kDa, low molecular weight hyaluronic acid having 200 kDa to 600 kDa, medium molecular weight hyaluronic acid having 600 kDa to 1,000 kDa, and high molecular weight hyaluronic acid having 800 kDa to 1,500 kDa, but is not limited thereto.

In one embodiment, the initial amount of hyaluronic acid of the present invention, the hyaluronic acid included in the composition for preparation, and/or the hyaluronic acid in the final formulation may have a concentration of 1 mg/mL to 100 mg/mL, specifically 1 mg/mL to 90 mg/mL, 1 mg/mL to 80 mg/mL, 1 mg/mL to 70 mg/mL, 1 mg/mL to 60 mg/mL, 1 mg/mL to 55 mg/mL, 1 mg/mL to 50 mg/mL, 1 mg/mL to 45 mg/mL, 1 mg/mL to 40 mg/mL, 1 mg/mL to 35 mg/mL, 1 mg/mL to 30 mg/mL, 1 mg/mL to 25 mg/mL, 1 mg/mL to 20 mg/mL, 1 mg/mL to 15 mg/mL, 1 mg/mL to 10 mg/mL, 10 mg/mL to 100 mg/mL, 10 mg/mL to 90 mg/mL, 10 mg/mL to 80 mg/mL, 10 mg/mL to 70 mg/mL, 10 mg/mL to 60 mg/mL, 10 mg/mL to 55 mg/mL, 10 mg/mL to 50 mg/mL, 10 mg/mL to 45 mg/mL, 10 mg/mL to 40 mg/mL, 10 mg/mL to 35 mg/mL, 10 mg/mL to 30 mg/mL, 10 mg/mL to 25 mg/mL, 10 mg/mL to 20 mg/mL, 10 mg/mL to 15 mg/mL, 15 mg/mL to 100 mg/mL, 15 mg/mL to 90 mg/mL, 15 mg/mL to 80 mg/mL, 15 mg/mL to 70 mg/mL, 15 mg/mL to 60 mg/mL, 15 mg/mL to 55 mg/mL, 15 mg/mL to 50 mg/mL, 15 mg/mL to 45 mg/mL, 15 mg/mL to 40 mg/mL, 15 mg/mL to 35 mg/mL, 15 mg/mL to 30 mg/mL, 15 mg/mL to 25 mg/mL, 15 mg/mL to 20 mg/mL, 20 mg/mL to 100 mg/mL, 20 mg/mL to 90 mg/mL, 20 mg/mL to 80 mg/mL, 20 mg/mL to 70 mg/mL, 20 mg/mL to 60 mg/mL, 20 mg/mL to 55 mg/mL, 20 mg/mL to 50 mg/mL, 20 mg/mL to 45 mg/mL, 20 mg/mL to 40 mg/mL, 20 mg/mL to 35 mg/mL, 20 mg/mL to 30 mg/mL, 20 mg/mL to 25 mg/mL, 25 mg/mL to 100 mg/mL, 25 mg/mL to 90 mg/mL, 25 mg/mL to 80 mg/mL, 25 mg/mL to 70 mg/mL, 25 mg/mL to 60 mg/mL, 25 mg/mL to 55 mg/mL, 25 mg/mL to 50 mg/mL, 25 mg/mL to 45 mg/mL, 25 mg/mL to 40 mg/mL, 25 mg/mL to 35 mg/mL, 25 mg/mL to 30 mg/mL, 30 mg/mL to 100 mg/mL, 30 mg/mL to 90 mg/mL, 30 mg/mL to 80 mg/mL, 30 mg/mL to 70 mg/mL, 30 mg/mL to 60 mg/mL, 30 mg/mL to 55 mg/mL, 30 mg/mL to 50 mg/mL, 30 mg/mL to 45 mg/mL, 30 mg/mL to 40 mg/mL, 30 mg/mL to 35 mg/mL, 50 mg/mL to 100 mg/mL, 50 mg/mL to 90 mg/mL, 50 mg/mL to 80 mg/mL, 50 mg/mL to 70 mg/mL, 50 mg/mL to 60 mg/mL, 15 mg/mL, 20 mg/mL, 25 mg/mL, or 50 mg/mL, but is not limited thereto.

In a specific example, the composition of the present invention may have various ranges of storage modulus that allow for treatment in various areas according to the molecular weight of the hyaluronic acid, even among ultra-low molecular weight, low molecular weight, medium molecular weight, and high molecular weight hyaluronic acids.

In one embodiment, the ornithine of the present invention may have a concentration of 0.5 wt% to 25 wt% relative to the initial amount of hyaluronic acid or the hyaluronic acid included in the composition for preparation. Specifically, it may have a concentration of 0.5 wt% to 25 wt%, 0.5 wt% to 21 wt%, 0.5 wt% to 15 wt%, 0.5 wt% to 10.5 wt%, 0.5 wt% to 10 wt%, 0.5 wt% to 8.7 wt%, 0.5 wt% to 5 wt%, 0.5 wt% to 4.5 wt%, 0.5 wt% to 4 wt%, 0.5 wt% to 3.5 wt%, 0.5 wt% to 3 wt%, 0.1 wt% to 2.5 wt%, 0.5 wt% to 2 wt%, 0.5 wt% to 1.7 wt%, 0.1 wt% to 1.5 wt%, 0.1 wt% to 1 wt%, 0.7 wt% to 25 wt%, 0.7 wt% to 21 wt%, 0.7 wt% to 15 wt%, 0.7 wt% to 10.5 wt%, 0.7 wt% to 10 wt%, 0.7 wt% to 8.7 wt%, 0.7 wt% to 5 wt%, 0.7 wt% to 3.5 wt%, 0.7 wt% to 1.7 wt%, 0.7 wt% to 1.5 wt%, 1.7 wt% to 25 wt%, 1.7 wt% to 21 wt%, 1.7 wt% to 15 wt%, 1.7 wt% to 10.5 wt%, 1.7 wt% to 10 wt%, 1.7 wt% to 8.7 wt%, 1.7 wt% to 5 wt%, 1.7 wt% to 3.5 wt%, 1.7 wt% to 1.5 wt%, 1 wt% to 25 wt%, 1 wt% to 21 wt%, 1 wt% to 20 wt%, 1 wt% to 15 wt%, 1 wt% to 10.5 wt%, 1 wt% to 10 wt%, 1 wt% to 8.7 wt%, 1 wt% to 5 wt%, 1 wt% to 4.5 wt%, 1 wt% to 4 wt%, 1 wt% to 3.5 wt%, 1 wt% to 3 wt%, 1 wt% to 2.5 wt%, 1 wt% to 2 wt%, 1 wt% to 1.7 wt%, 1 wt% to 1.5 wt%, 2 wt% to 25 wt%, 2 wt% to 21 wt%, 2 wt% to 20 wt%, 2 wt% to 15 wt%, 2 wt% to 10.5 wt%, 2 wt% to 10 wt%, 2 wt% to 8.7 wt%, 2 wt% to 5 wt%, 2 wt% to 4.5 wt%, 2 wt% to 4 wt%, 2 wt% to 3.5 wt%, 2 wt% to 3 wt%, 2 wt% to 2.5 wt%, 3.5 wt% to 25 wt%, 3.5 wt% to 21 wt%, 3.5 wt% to 15 wt%, 3.5 wt% to 10.7 wt%, 3.5 wt% to 10 wt%, 3.5 wt% to 8.7 wt%, 3.5 wt% to 5 wt%, 3.5 wt% to 4.5 wt%, 3.5 wt% to 4 wt%, 5 wt% to 25 wt%, 5 wt% to 20 wt%, 5 wt% to 15 wt%, or 5 wt% to 10 wt%, but is not limited thereto.

In one embodiment, the STMP of the present invention may have a concentration of 1 wt% to 130 wt% relative to the initial amount of hyaluronic acid or the hyaluronic acid included in the composition for preparation. Specifically, it may have a concentration of 1 wt% to 150 wt%, 1 wt% to 130 wt%, 1 wt% to 127 wt%, 1 wt% to 100 wt%, 1 wt% to 63.7 wt%, 1 wt% to 50 wt%, 1 wt% to 40 wt%, 1 wt% to 38 wt%, 1 wt% to 35 wt%, 1 wt% to 30 wt%, 1 wt% to 25 wt%, 1 wt% to 20 wt%, 1 wt% to 12.7 wt%, 1 wt% to 10 wt%, 1 wt% to 5 wt%, 1 wt% to 2.5 wt%, 1 wt% to 1.25 wt%, 1.25 wt% to 150 wt%, 1.25 wt% to 130 wt%, 1.25 wt% to 127 wt%, 1.25 wt% to 100 wt%, 1.25 wt% to 63.7 wt%, 1.25 wt% to 50 wt%, 1.25 wt% to 40 wt%, 1.25 wt% to 38 wt%, 1.25 wt% to 35 wt%, 1.25 wt% to 30 wt%, 1.25 wt% to 25 wt%, 1.25 wt% to 20 wt%, 1.25 wt% to 12.7 wt%, 1.25 wt% to 10 wt%, 1.25 wt% to 5 wt%, 1.25 wt% to 2.5 wt%, 2.5 wt% to 150 wt%, 2.5 wt% to 130 wt%, 2.5 wt% to 127 wt%, 2.5 wt% to 100 wt%, 2.5 wt% to 63.7 wt%, 2.5 wt% to 50 wt%, 2.5 wt% to 40 wt%, 2.5 wt% to 38 wt%, 2.5 wt% to 35 wt%, 2.5 wt% to 30 wt%, 2.5 wt% to 25 wt%, 2.5 wt% to 20 wt%, 2.5 wt% to 12.7 wt%, 2.5 wt% to 10 wt%, 2.5 wt% to 5 wt%, 5 wt% to 150 wt%, 5 wt% to 130 wt%, 5 wt% to 127 wt%, 5 wt% to 100 wt%, 5 wt% to 63.7 wt%, 5 wt% to 50 wt%, 5 wt% to 40 wt%, 5 wt% to 38 wt%, 5 wt% to 35 wt%, 5 wt% to 30 wt%, 5 wt% to 25 wt%, 5 wt% to 20 wt%, 5 wt% to 12.7 wt%, 5 wt% to 10 wt%, 10 wt% to 150 wt%, 10 wt% to 130 wt%, 10 wt% to 127 wt%, 10 wt% to 100 wt%, 10 wt% to 63.7 wt%, 10 wt% to 50 wt%, 10 wt% to 40 wt%, 10 wt% to 38 wt%, 10 wt% to 35 wt%, 10 wt% to 30 wt%, 10 wt% to 25 wt%, 10 wt% to 20 wt%, 12.7 wt% to 150 wt%, 12.7 wt% to 130 wt%, 12.7 wt% to 127 wt%, 12.7 wt% to 100 wt%, 12.7 wt% to 63.7 wt%, 12.7 wt% to 50 wt%, 12.7 wt% to 40 wt%, 12.7 wt% to 38 wt%, 12.7 wt% to 35 wt%, 12.7 wt% to 30 wt%, 12.7 wt% to 25 wt%, 12.7 wt% to 20 wt%, 25 wt% to 150 wt%, 25 wt% to 130 wt%, 25 wt% to 127 wt%, 25 wt% to 100 wt%, 25 wt% to 63.7 wt%, 25 wt% to 50 wt%, 25 wt% to 40 wt%, 25 wt% to 38 wt%, 25 wt% to 35 wt%, 25 wt% to 30 wt%, 38 wt% to 150 wt%, 38 wt% to 130 wt%, 38 wt% to 127 wt%, 38 wt% to 100 wt%, 38 wt% to 63.7 wt%, 38 wt% to 50 wt%, 38 wt% to 45 wt%, 50 wt% to 150 wt%, 50 wt% to 130 wt%, 50 wt% to 127 wt%, 50 wt% to 100 wt%, or 50 wt% to 63.7 wt%, but is not limited thereto.

The molecular weight of the hyaluronic acid and the concentrations of hyaluronic acid, STMP, and ornithine may refer to the molecular weight and concentration of the components included in the initial amount or the composition in the method according to the present invention.

In one embodiment, the crosslinked hyaluronic acid may be capable of controlling the concentrations of hyaluronic acid, ornithine, and STMP according to the desired storage modulus.

It may be important to control the storage modulus of the crosslinked hyaluronic acid of the present invention such that a desired filler can be properly injected into a desired tissue. Specifically, it may exhibit an elastic modulus of about 100 Pa to about 1,000 Pa, specifically about 100 Pa to about 800 Pa, so that it can be used in various regions (tissues).

In one embodiment, for the crosslinked hyaluronic acid to have the desired storage modulus, ornithine may be included in an amount of 0.5 wt% to 25 wt% relative to the amount of hyaluronic acid added, and STMP may be included in an amount of 1 wt% to 130 wt% relative to the amount of hyaluronic acid added. Specifically, ornithine may be included in an amount of 0.7 wt% to 21 wt% relative to the amount of hyaluronic acid added, and STMP may be included in an amount of 1.25 wt% to 127 wt% relative to the amount of hyaluronic acid added.

In one embodiment, for the crosslinked hyaluronic acid to have the desired storage modulus, ornithine may be included in an amount of 0.7 wt% to 21 wt% relative to the amount of hyaluronic acid added, and STMP may be included in an amount of 38 wt% relative to the amount of hyaluronic acid added.

In one example, when ornithine is included in an amount of 0.7 wt% to 21 wt% relative to the amount of hyaluronic acid added and STMP is included in an amount of 38 wt% relative to the amount of hyaluronic acid added, the crosslinked hyaluronic acid of the present invention may have a storage modulus range of about 100 Pa or more, specifically about 100 Pa to about 1,000 Pa; when ornithine is included in an amount of 1.7 wt% to 10.5 wt% relative to the amount of hyaluronic acid added and STMP is included in an amount of 38 wt% relative to the amount of hyaluronic acid added, the crosslinked hyaluronic acid of the present invention may have a storage modulus range of about 300 Pa or more, specifically about 300 Pa to about 1,000 Pa; when ornithine is included in an amount of 3.5 wt% to 10.5 wt% relative to the amount of hyaluronic acid added and STMP is included in an amount of 38 wt% relative to the amount of hyaluronic acid added, the crosslinked hyaluronic acid of the present invention may have a storage modulus range of about 400 Pa or more, specifically about 400 Pa to about 1,000 Pa; when ornithine is included in an amount of 3.5 wt% to 8.7 wt% relative to the amount of hyaluronic acid added and STMP is included in an amount of 38 wt% relative to the amount of hyaluronic acid added, the crosslinked hyaluronic acid of the present invention may have a storage modulus range of about 500 Pa or more, specifically about 500 Pa to about 1,000 Pa; and when ornithine is included in an amount of 8.7 wt% relative to the amount of hyaluronic acid added and STMP is included in an amount of 38 wt% relative to the amount of hyaluronic acid added, the crosslinked hyaluronic acid of the present invention may have a storage modulus range of about 700 Pa or more, specifically about 700 Pa to about 1,000 Pa, but is not limited thereto.

In one embodiment, when ornithine is included in an amount of 8.7 wt% relative to the amount of hyaluronic acid added, STMP may be included in an amount of 1.25 wt% to 127 wt% relative to the amount of hyaluronic acid added for the crosslinked hyaluronic acid to have the desired storage modulus.

In one example, when ornithine is included in an amount of 8.7 wt% relative to the amount of hyaluronic acid added and STMP is included in an amount of 1.25 wt% to 127 wt% relative to the amount of hyaluronic acid added, the crosslinked hyaluronic acid of the present invention may have a storage modulus range of about 600 Pa or more, specifically about 600 Pa to about 1,000 Pa; when ornithine is included in an amount of 8.7 wt% relative to the amount of hyaluronic acid added and STMP is included in an amount of 2.5 wt% to 63.7 wt% relative to the amount of hyaluronic acid added, the crosslinked hyaluronic acid of the present invention may have a storage modulus range of about 650 Pa or more, specifically about 650 Pa to about 1,000 Pa; when ornithine is included in an amount of 8.7 wt% relative to the amount of hyaluronic acid added and STMP is included in an amount of 12.7 wt% to 63.7 wt% relative to the amount of hyaluronic acid added, the crosslinked hyaluronic acid of the present invention may have a storage modulus range of about 670 Pa or more, specifically about 670 Pa to about 1,000 Pa; when ornithine is included in an amount of 8.7 wt% relative to the amount of hyaluronic acid added and STMP is included in an amount of 63.7 wt% relative to the amount of hyaluronic acid added, the crosslinked hyaluronic acid of the present invention may have a storage modulus range of about 700 Pa or more; and when ornithine is included in an amount of 8.7 wt% relative to the amount of hyaluronic acid added and STMP is included in an amount of 38 wt% relative to the amount of hyaluronic acid added, the crosslinked hyaluronic acid of the present invention may have a storage modulus range of about 750 Pa or more, but is not limited thereto.

Specifically, it can be regarded that controlling the concentrations of ornithine and STMP is important for the crosslinked hyaluronic acid of the present invention to exhibit the desired storage modulus.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. For example, the term "about" can refer to a range of -10% to +10% of the numerical value. As another example, the term "about" may refer to a range of -5% to +5% of the given numerical value. However, it is not limited thereto.

In addition, the crosslinked hyaluronic acid of the present invention may not have hyaluronic acid, ornithine, and STMP repeated in a constant repeating unit. Specifically, the crosslinked hyaluronic acid of the present invention may comprise one or more monomeric units of Chemical Formulas 3 to 7 below, but the monomeric units may be irregularly repeated rather than being repeated in a constant repeating unit.

In one embodiment, the crosslinked hyaluronic acid of the present invention may comprise one or more, two or more, three or more, four or more, or five types of monomeric units of Chemical Formulas 3 to 7 below, and may be a composite composition.

Chemical Formulas 3 to 7 below merely correspond to examples of monomeric units constituting the crosslinked hyaluronic acid of the present invention, and the crosslinked hyaluronic acid of the present invention is not limited to any specific structure as long as it is double-crosslinked with ornithine and STMP.

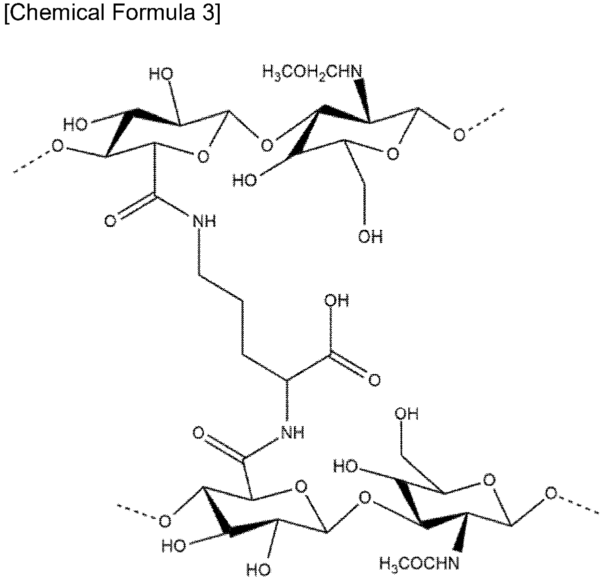

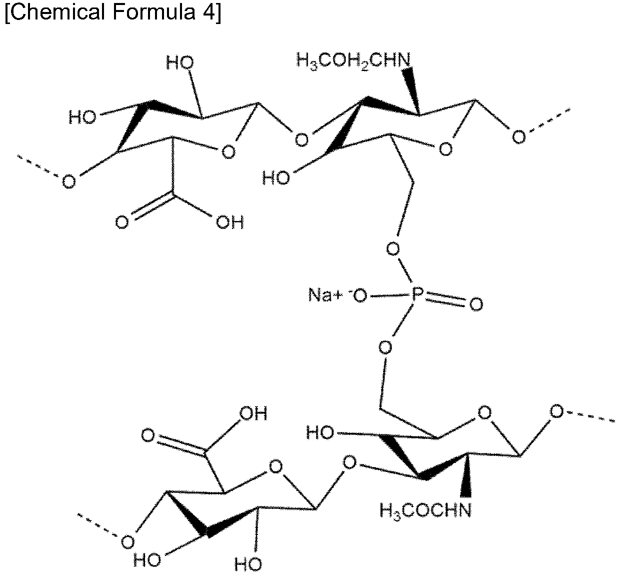

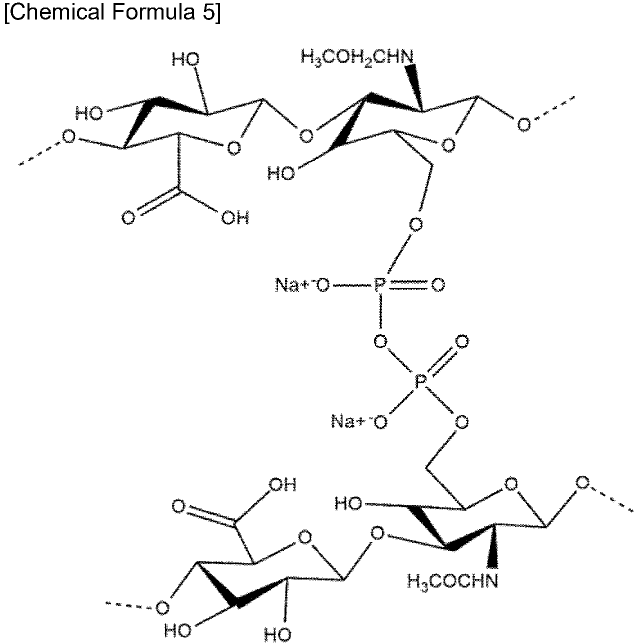

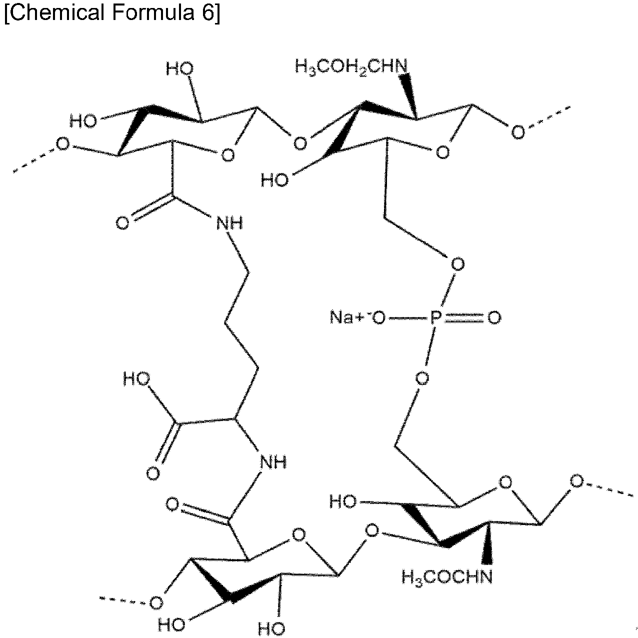

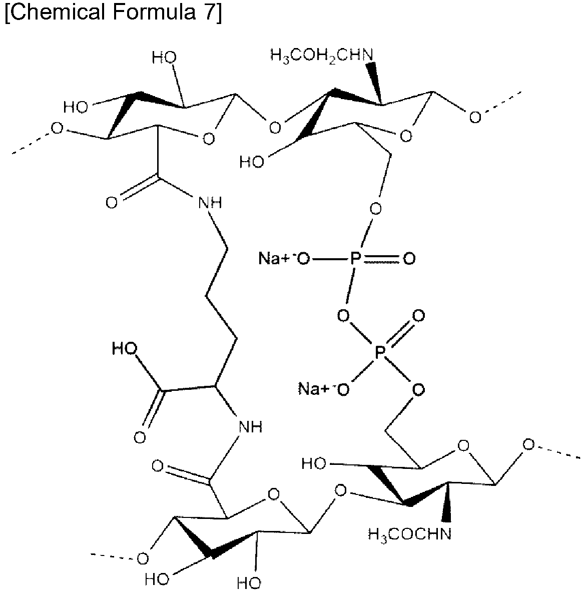

As used herein, the term "double-crosslinked hyaluronic acid" refers to hyaluronic acid that is crosslinked with both ornithine and STMP, rather than one in which ornithine and STMP are crosslinked to each other.

In one example of the present invention, the STMP and ornithine may be safe as they do not exhibit cytotoxicity compared to divinyl sulfone (DVS) and 1,4-butanediol diglycidyl ether (BDDE), which are widely used in hyaluronic acid crosslinking.

In one embodiment, the crosslinked hyaluronic acid of the present invention may have excellent storage modulus and thermal stability while maintaining low cytotoxicity compared to hyaluronic acid crosslinked with STMP alone and hyaluronic acid crosslinked with ornithine alone, but is not limited thereto.

In addition, the crosslinked hyaluronic acid of the present invention has excellent biocompatibility and can be used as a composition for tissue repair, *in vivo* administration, and fillers.

Still another aspect of the present invention provides a composition for tissue repair, comprising the crosslinked hyaluronic acid of the present invention.

The crosslinked hyaluronic acid is as described in the aspects above.

In the present invention, the term "for tissue repair" means that it may be used for the replacement, repair, and/or reconstruction of human tissues and organs, such as blood vessels, the heart, diaphragms, fascia, and/or skin.

Still another aspect of the present invention provides a filler composition, comprising the crosslinked hyaluronic acid of the present invention.

The crosslinked hyaluronic acid is as described in the aspects above.

In the present invention, the term "filler" refers to a medical device that is injected into the skin for volume restoration or the like as one of the tissue repair materials and functions to maintain skin volume through physical repair.

In one embodiment, the filler composition of the present invention may be a composition for dermal fillers.

In one embodiment, the filler composition of the present invention may be a hyaluronic acid filler composition. Hyaluronic acid fillers consist of polysaccharides similar to the components of the human body, resulting in fewer side effects including skin hypersensitivity reactions. They are easy to administer and remove, exhibit excellent storage modulus, and maintain skin moisture, volume, and storage modulus, making them currently the most widely used fillers. Generally, when manufacturing hyaluronic acid fillers, crosslinking agents are used to ensure that hyaluronic acid remains in the body without degrading for a long time. However, divinyl sulfone (DVS) and 1,4-butanediol diglycidyl ether (BDDE), which are widely used as hyaluronic acid crosslinking agents, have the drawback of exhibiting biological toxicity.

In one embodiment, the filler composition of the present invention may use ornithine and STMP as crosslinking agents, which exhibit significantly low cytotoxicity compared to divinyl sulfone (DVS) and 1,4-butanediol diglycidyl ether (BDDE), thereby providing excellent biocompatibility.

The filler composition of the present invention may have superior storage modulus and thermal stability compared to a filler composition comprising hyaluronic acid crosslinked with STMP alone and hyaluronic acid crosslinked with ornithine alone, but is not limited thereto.

Still another aspect of the present invention provides a composition for *in vivo* administration, comprising the crosslinked hyaluronic acid of the present invention.

The crosslinked hyaluronic acid is as described in the aspects above.

In the present invention, the term "administration" means introducing the composition of the present invention into a subject by an appropriate method, and the composition may be administered through various routes of administration, such as topical application, subcutaneous administration, or dermal application, as long as the target tissue can be reached. The formulation may be a topical, subcutaneous, dermal, or intramuscular formulation, but is not limited thereto. The preferred dosage of the composition of the present invention may vary depending on the condition of the subject.

The composition for tissue repair, filler composition, and composition for *in vivo* administration according to the present invention may further comprise one or more compounds selected from the group consisting of anesthetics, polyols, vitamins, amino acids, metals, antioxidants, and mineral salts, and may include a buffer solution, such as a phosphate buffer, to adjust the pH.

The composition for tissue repair, filler composition, and composition for *in vivo* administration of the present invention can provide an injectable composition. The term "injectable" refers to a material having the characteristics required for administering the composition into the dermal region of a subject using an injection device equipped with a fine needle.

The crosslinked hyaluronic acid, composition for tissue repair, filler composition, and/or composition for *in vivo* administration of the present invention may exhibit an elastic modulus within an appropriate range for injection into tissues, but are not limited thereto.

As used herein, the term "elasticity" refers to the property of a solid that is observed when an external force is applied to the object-that is, the property of deforming under applied force but returning to its original shape upon removal of the force. In one example, the elasticity of a filler indicates the degree to which the filler maintains a desired shape under skin tissue, and the higher the elasticity, the longer the initially formed shape is maintained. Such elasticity can be expressed as the elastic modulus (storage modulus; G'), and the unit may be Pascal (Pa).

The higher the storage modulus of crosslinked hyaluronic acid or a composition comprising the same, the more it forms a particle-like structure that feels slightly firm, thereby better providing volume and maintaining its shape after a single administration. In addition, although the duration of the filler increases as storage modulus increases, if the storage modulus is excessively high, the surface is uneven, which may result in the problem of forming layers on the skin depending on the skill level.

In one embodiment, the crosslinked hyaluronic acid, composition for tissue repair, filler composition, and/or composition for *in vivo* administration of the present invention may exhibit an elastic modulus of about 100 Pa to 1,000 Pa, specifically 100 Pa to 800 Pa. In one example, hydrogels with a low storage modulus of 100 Pa or less may form unstable crosslinks, and excessively hydrogels with a high storage modulus of 800 Pa or more may cause foreign body reactions in the human body.

Among the above, those having a low to medium elastic modulus can be used for the lips, facial contouring, skin improvement, mid-face, and the like; those having a medium elastic modulus can be used for the lower face and the like; and those having a medium to high elastic modulus can be used for the upper face, mid-face, nose, chin, and the like. As such, the required characteristics may vary according to the region of administration, and accordingly, hydrogels may have different ranges of elastic moduli (Carola de la Guardia *et al* ., 2022;38:116-123; and Won Lee *et al* ., 2019; 00:1-9, doi:10.1097/dss.0000000000001858).

Therefore, it may be important to control the storage modulus according to the target tissue to inject the desired filler.

Still another aspect of the present invention provides a composition for producing crosslinked hyaluronic acid, a composition for producing biomaterial for tissue repair, a composition for producing a filler, and a composition for producing *in vivo* administration agent, comprising ornithine, STMP, and hyaluronic acid.

The ornithine, STMP, hyaluronic acid, crosslinked hyaluronic acid, for tissue repair, filler, and the like are as described in the aspects above.

The composition of the present invention may further comprise a suitable excipient commonly used in the production of biomaterials for crosslinked hyaluronic acid, fillers, or tissue repair, and/or composition for *in vivo* administration. Examples of such excipients include preservatives, wetting agents, dispersing agents, suspending agents, buffering agents, stabilizers, or isotonic agents, but the excipient is not limited thereto.

Still another aspect of the present invention provides a kit for producing crosslinked hyaluronic acid, a composition for tissue repair, a filler composition, and a composition for *in vivo* administration, comprising ornithine, STMP, and hyaluronic acid.

The ornithine, STMP, hyaluronic acid, crosslinked hyaluronic acid, and the like are as described in the aspects above.

The kit may be provided in a form that stores hyaluronic acid, ornithine, and STMP in separate containers so that they can be mixed at the time of use, but is not limited thereto.

Additionally, the kit of the present invention may comprise one or more other components, compositions, solutions, or devices, for the preparation of crosslinked hyaluronic acid, a composition for tissue repair, a filler composition, and a composition for *in vivo* administration, but is not limited thereto. Examples of the other components may include suitable carriers, solvents, buffers, stabilizers, but are not limited thereto. The carrier may include soluble carriers and insoluble carriers. Examples of soluble carriers include physiologically acceptable buffers known in the art, such as PBS, and examples of insoluble carriers may include polymers such as polystyrene, polyethylene, polypropylene, polyester, polyacrylonitrile, fluoropolymer, crosslinked dextran, polysaccharide, and latex with metal plated, as well as other paper, glass, metal, agarose, and combinations thereof, but the carriers are not limited thereto.

In addition, the kit of the present invention may further comprise a user guide describing the optimal conditions for carrying out the reaction. The guide is a printed document that describes how to use the kit, for example, the reaction conditions and the like. The guide includes instruction booklets in the form of pamphlets or flyers, labels attached to the kit, and descriptions on the surface of the package comprising the kit. In addition, the guide includes information disclosed or provided through electronic media such as the Internet.

Still another aspect of the present invention provides a method for improving skin, comprising administering the crosslinked hyaluronic acid, composition for tissue repair, the filler composition, and/or the composition for *in vivo* administration of the present invention.

The crosslinked hyaluronic acid, composition, subject, and administration are as described in the aspects above.

The skin improvement may include skin filling, (soft) tissue repair, wrinkle improvement, and skin volume augmentation, but is not limited thereto.

As used herein, the term "subject" refers to any animal requiring skin improvement, and may refer to all mammals including dogs, cattle, horses, rabbits, mice, rats, chickens, and humans.

Still another aspect of the present invention provides a composite composition comprising one or more monomeric units of Formulas 3 to 7.

In addition, the composite composition of the present invention may comprise one or more monomeric units of Chemical Formulas 3 to 7 above that are irregularly repeated rather than being repeated in a constant repeating unit.

In one embodiment, the composite composition of the present invention may comprise one or more, two or more, three or more, four or more, or five types of monomeric units of Chemical Formulas 3 to 7 above. However, Chemical Formulas 3 to 7 are merely examples of the monomeric unit of the present invention, and thus the monomeric unit is not limited thereto.

In one embodiment, the composite composition may comprise directly or indirectly connected monomeric units, but is not limited thereto.

In one embodiment, the composite composition may be used as a composition for tissue repair, a filler composition, and/or a composition for *in vivo* administration.

### [Mode for Carrying Out the Invention]

The present invention will be described in detail by way of Examples. However, the following Examples are merely preferred embodiments for illustrating the present invention and are not intended to limit the scope of the rights of the present invention thereto. Meanwhile, the technical descriptions absent in the present disclosure may be sufficiently understood and readily practiced by those skilled in the art of the present invention or related art.

### Example 1. Biocompatibility evaluation according to the type of crosslinking agent

The biocompatibility of various crosslinking agents was evaluated by cytotoxicity using a known method (Chang Hee Jeong, et al., 2021, Toxicology In Vitro Feb;70:105034; ISO10993-5). The negative/positive control groups and experimental groups are as shown in Table 1 below. Divinyl sulfone (DVS) and 1,4-butanediol diglycidyl ether (BDDE), used as experimental groups, are existing crosslinking agents that are widely used. In the present invention, biocompatibility was evaluated to determine whether ornithine and STMP are suitable crosslinking agents.

**[Table 1]**

| Control group | Negative control group: high-density polyethylene film | | | | | |
|---|---|---|---|---|---|---|
| | Positive control group: polyurethane film containing 0.1% zinc diethyldithiocarbamate (ZDEC) | | | | | |
| Experimental groups (DVS, BDDE, ornithine, STMP, ornithine+STMP) | 0 ppm | 5 ppm | 25 ppm | 100 ppm | 500 ppm | 1,000 ppm |

Specifically, human dermal fibroblast (HDF) was seeded into a 96-well plate at a cell density of 1 X 10⁴ cells/well and cultured for 24 hours in a 37°C, 5% CO₂ incubator. Subsequently, the control and experimental group samples of Table 1 were treated with 0 to 1,000 ppm and cultured for 24 hours in a 37°C and 5% CO₂ incubator, then treated with WST reagent and reacted for 1 hour in a 37°C and 5% CO₂ incubator, after which the absorbance (OD₄₅₀) was measured at 450 nm. Cell viability was determined as follows, and if cell viability decreased by more than 30% depending on the treatment of the experimental group and the control group, the sample was regarded as having cytotoxicity.
- $Viability \left(%\right) = \left(Exp.-Blank\right) / \left(Control-Blank\right) \times 100$

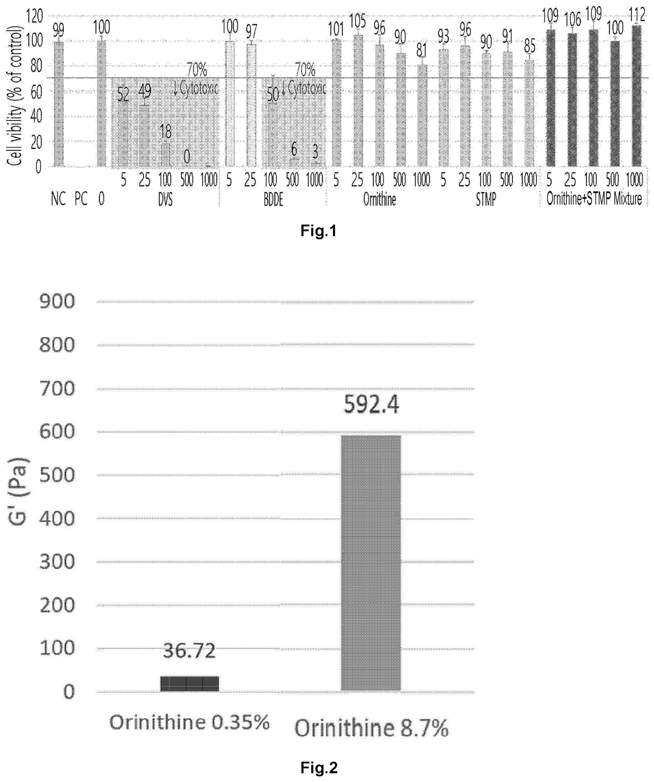

As a result, as shown in FIG. 1, the conventionally used known crosslinking agent DVS showed cytotoxicity from 5 ppm, and BDDE from 100 ppm, while ornithine alone and STMP alone did not show cytotoxicity up to 1,000 ppm. In addition, the mixture of ornithine and STMP did not show cytotoxicity up to 1,000 ppm.

Accordingly, it was confirmed that ornithine, STMP, and mixtures of ornithine and STMP exhibit higher biocompatibility compared to DVS and BDDE, which are conventionally used crosslinking agents. Based on the above results, the present invention conducted experiments using ornithine, STMP, and ornithine and STMP as crosslinking agents.

### Example 2. Preparation and results of hyaluronic acid formulation crosslinked with ornithine alone

### Example 2-1. Preparation of hyaluronic acid formulation crosslinked with ornithine alone

Hyaluronic acid (HA) crosslinked with ornithine alone was prepared.

In particular, hyaluronic acid with a molecular weight of 20 kDa to 2,000 kDa and ornithine with an initial concentration of 0.35 wt% to 8.7 wt% relative to hyaluronic acid were used. To prepare hyaluronic acid crosslinked with ornithine alone, hyaluronic acid and ornithine were dissolved at pH 4 to 6, and N-hydroxysuccinimide (NHS) was added and mixed for 30 minutes. Thereafter, 1-ethyl-3-(3-dimethylaminopropyl)carbodiimide (EDC) was added, followed by mixing and crosslinking for 3 hours. NaOH solution was added to break the ester bonds, and HCl solution was added for neutralization. Then, dialysis was performed in PBS for 4 days. Uncrosslinked hyaluronic acid (uncrosslinked HA) and lidocaine were added or not added, and grinding and final concentration adjustment were performed.

### Example 2-2. Confirmation of Storage modulus

The phase angle is used to analyze the degree of storage modulus of a material, and the detailed analysis conditions for the phase angle used in the experiment are as shown in Table 2.

**[Table 2]**

| Oscillation (1.0 Hz) | |
|---|---|
| Geometry | Parallel Plate 20 mm |
| Loading gap | 1.0 mm |
| Frequency | 0.1 Hz to 10 Hz |

A phase angle of 0° represents an elastic material (i.e., solid), and a phase angle of 90° represents a viscous material (i.e., liquid). To be used as a filler, the phase angle must be 80° or less (WO 2016-096920 A1). The storage modulus was confirmed based on ornithine concentration. Specifically, storage modulus was confirmed at ornithine concentrations of 0.35 wt% to 8.7 wt%.

As a result, as shown in FIG. 2, it was confirmed that the storage modulus was higher when the ornithine concentration was 8.7 wt%.

### Example 3. Preparation and results of hyaluronic acid formulation crosslinked with STMP alone

### Example 3-1. Preparation of hyaluronic acid formulation crosslinked with STMP alone

Hyaluronic acid (HA) crosslinked with STMP alone was prepared.

In particular, hyaluronic acid with a molecular weight of 20 kDa to 2,000 kDa was used. To prepare hyaluronic acid crosslinked with STMP alone (WO 2014/181147 A1), hyaluronic acid dissolved in a 0.25 M NaOH solution for 2.5 hours and an STMP solution dissolved in a 0.25 M NaOH solution were mixed for 30 minutes. Then, a crosslinking reaction was performed at 50°C for 15 hours.

### Example 3-2. Confirmation of cross-linking reaction progress

After the addition of STMP, the pH decreased as the cross-linking reaction proceeded, and the pH measured after the completion of the cross-linking reaction was 8.58. As crosslinking progresses, free phosphate groups are generated, causing the pH to decrease; therefore, a decrease in pH indicates that the crosslinking reaction is in progress.

### Example 3-3. Confirmation of non-formation of a stable hydrogel structure

It was identified whether the STMP forms a stable hydrogel structure using the phase angle under the same conditions as the experiment in Example 2-2.

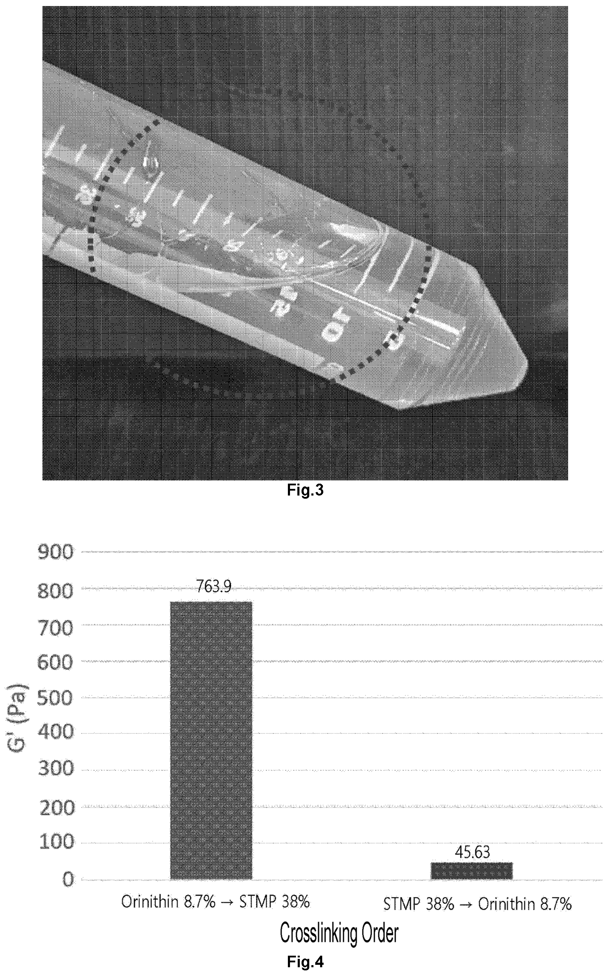

As a result, in the case of hyaluronic acid crosslinked with STMP alone, the phase angle was 84.9°, confirming that it did not form a stable hydrogel structure (FIG. 3). The results suggest that tissue repair may not be possible by crosslinking with STMP alone. Therefore, hyaluronic acid crosslinked with STMP alone did not form a stable hydrogel structure, and therefore, the subsequent steps such as the neutralization step with HCl were not carried out.

### Example 4. Preparation and results of hyaluronic acid formulation double-crosslinked with ornithine and STMP

### Example 4-1. Preparation of hyaluronic acid formulation double-crosslinked with ornithine and STMP

Double-crosslinked hyaluronic acid was prepared by crosslinking with sodium trimetaphosphate (STMP) followed by ornithine to verify the effect of the order of crosslinking ornithine and STMP.

In particular, hyaluronic acid with a molecular weight of 20 kDa to 2,000 kDa, ornithine with an initial concentration of 0.1 wt% to 15 wt% relative to hyaluronic acid, and STMP with an initial concentration of 0.1 wt% to 250 wt% relative to hyaluronic acid were used.

### Example 4-1-1. Preparation of double-crosslinked hyaluronic acid formulation by crosslinking with STMP followed by crosslinking with ornithine

To prepare double-crosslinked hyaluronic acid by crosslinking with STMP followed by crosslinking with ornithine, hyaluronic acid was dissolved at pH 10 to 12, and STMP was added then dissolved.

The mixture was mixed and crosslinked at room temperature for 30 minutes or more. HCl solution was added to the STMP-crosslinked product to lower the pH to 4 to 6. Then, ornithine and N-hydroxysuccinimide (NHS) were added and mixed for 30 minutes or more, after which 1-ethyl-3-(3-dimethylaminopropyl)carbodiimide (EDC) was additionally added, followed by further mixing and crosslinking for 30 minutes or more. NaOH solution was added to cleave unwanted ester crosslinks, and HCl solution was added for neutralization. Dialysis was performed in PBS for more than 3 days, and uncrosslinked hyaluronic acid (uncrosslinked HA) and lidocaine were added or not added. Then, grinding and the final concentration adjustment were performed.

### Example 4-1-2. Preparation of double-crosslinked hyaluronic acid formulation by crosslinking with ornithine followed by crosslinking with STMP

To prepare double-crosslinked hyaluronic acid by crosslinking with ornithine followed by crosslinking with STMP, hyaluronic acid and ornithine were dissolved at pH 4 to 6, and NHS (N-hydroxysuccinimide) was added and mixed for 30 minutes or more. Thereafter, 1-ethyl-3-(3-dimethylaminopropyl)carbodiimide (EDC) was added, followed by mixing and crosslinking for 30 minutes or more. NaOH solution was added and mixed for 30 minutes or more to cleave unwanted ester crosslinks, and STMP was added for crosslinking. STMP crosslinking was carried out for 30 minutes or more, and HCl solution was added for neutralization. Dialysis was performed in PBS for more than 3 days, and uncrosslinked hyaluronic acid (uncrosslinked HA) and lidocaine were added or not added. Then, grinding and the final concentration adjustment were performed.

### Example 4-2. Analysis of effects of crosslinking order of ornithine and STMP on storage modulus

In order to confirm the effect of the crosslinking order of ornithine and STMP on storage modulus, crosslinking with ornithine was performed after crosslinking with STMP under the conditions of an ornithine concentration of 8.7 wt% and an STMP concentration of 38 wt%, which provided the most superior storage modulus. As a result, as shown in FIG. 4, when STMP was crosslinked first, the storage modulus of the final product was very low, indicating that the crosslinked product was not well formed.

This suggests that the double-crosslink of the present invention can produce crosslinked hyaluronic acid with a diverse range of storage modulus when carried out in the order of crosslinking with ornithine followed by crosslinking with STMP.

### Example 4-3. Analysis of effects of ornithine concentration on storage modulus

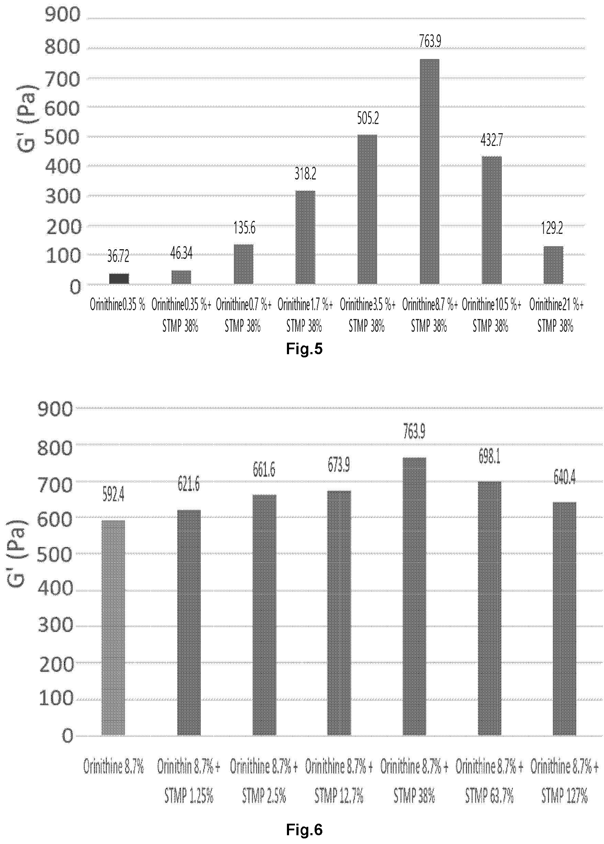

Storage modulus analysis was performed after double crosslinking was performed by crosslinking with ornithine followed by crosslinking with STMP. Crosslinking was performed by using ornithine at various concentrations while fixing the STMP concentration at 38 wt%, and the storage modulus was analyzed. As shown in FIG. 5, it was confirmed that the products have a diverse elastic range (approximately 100 Pa to 800 Pa) that allows for treatment in various areas.

Specifically, it was confirmed that ornithine could be included at 0.7 wt% to 21 wt% relative to the amount of hyaluronic acid added and STMP at 38 wt% relative to the amount of hyaluronic acid added to produce crosslinked hyaluronic acid with a storage modulus of about 100 Pa. In addition, it was confirmed that ornithine could be included at 1.7 wt% relative to the amount of hyaluronic acid added and STMP at 38 wt% relative to the amount of hyaluronic acid added to produce crosslinked hyaluronic acid with a storage modulus of about 300 Pa. It was confirmed that ornithine could be included at 8.7 wt% relative to the amount of hyaluronic acid added and STMP at 1.25 wt% to 127 wt% relative to the amount of hyaluronic acid added to produce crosslinked hyaluronic acid with a storage modulus of about 600 Pa.

### Example 4-4. Analysis of effects of STMP concentration on storage modulus

Based on the results of Example 2-2, the effect of STMP concentration on storage modulus was identified when the ornithine concentration was 8.7 wt%.

As a result, as shown in FIG. 6, it was confirmed that the storage modulus increased when double-crosslinked with ornithine and STMP compared to crosslinking with ornithine alone, and that the storage modulus showed the highest rate of increase at 38 wt% of STMP. In particular, it was confirmed that when the concentration of ornithine is fixed at 8.7 wt%, detailed control of storage modulus is possible by varying the concentration of STMP, thereby increasing the storage modulus from 29.2 Pa to 71.5 Pa compared to crosslinking with 8.7 wt% of ornithine alone, so that the final storage modulus value may be approximately 600 Pa to 800 Pa.

This suggests that the concentrations of ornithine and STMP can be controlled to produce the crosslinked hyaluronic acid of the present invention to have the desired storage modulus, and in particular, the specific value of storage modulus can be controlled by controlling the concentration of STMP.

### Example 5. Storage modulus experiment according to hyaluronic acid molecular weight

It was confirmed that the hyaluronic acid double-crosslinked with ornithine and STMP produced by the method of the present invention exhibited a diverse range of storage modulus suitable for procedures in various regions, and it was determined whether it exhibits an appropriate range of storage modulus suitable for procedures even at various molecular weights of hyaluronic acid.

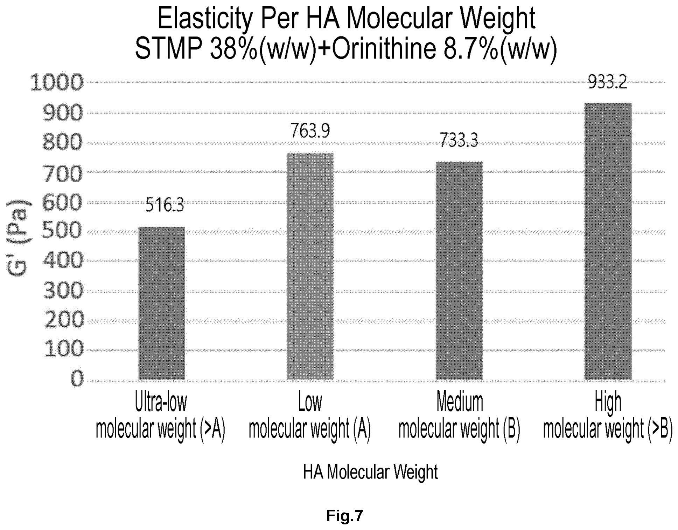

As a result, as shown in FIG. 7, it was confirmed that ultra-low molecular weight (approx. 25 kDa), low molecular weight (approx. 435 kDa), medium molecular weight (approx. 800 kDa), and high molecular weight (approx. 1140 kDa) hyaluronic acid have a variety of storage modulus ranges that allow for treatment in various regions.

This suggests that even when hyaluronic acid of different molecular weights is applied, crosslinked hyaluronic acid with a diverse range of storage modulus suitable for procedures in various regions is well formed.

The above results suggest that the present invention has excellent effects in enabling the production of filler compositions, compositions for *in vivo* administration, *etc*., that exhibit excellent biocompatibility and physical properties in various parts of the body.

From the above description, those skilled in the art to which the present invention pertains would understand that the present invention can be implemented in other specific forms without changing its technical concept or essential features. Therefore, the embodiments described above should be construed as being exemplified and not limiting the present disclosure. The scope of the present invention should be interpreted to include all modifications or modified forms derived from the meaning and scope of the claims as set forth below, as well as equivalent concepts, rather than from the detailed description above.

## Claims

1. A method for producing crosslinked hyaluronic acid, comprising:
step (a) of crosslinking hyaluronic acid with ornithine; and
step (b) of crosslinking the hyaluronic acid crosslinked with ornithine in step (a) with sodium trimetaphosphate (STMP).

2. The method of claim 1, wherein the ornithine has a concentration of 0.5 wt% to 25 wt% relative to the amount of hyaluronic acid added.

3. The method of claim 1, wherein the STMP has a concentration of 1 wt% to 130 wt% relative to the amount of hyaluronic acid added.

4. The method of claim 1, comprising one or more of: a step of dissolving hyaluronic acid and ornithine in a buffer prior to step (a); and a step of adding a coupling activator.

5. The method of claim 1, further comprising, between step (a) and step (b), one or more of: a step of adding a basic solution to cleave unwanted ester crosslinks between hyaluronic acids; a paste step; and a step of adding STMP.

6. The method of claim 1, further comprising, after step (b), one or more of: a step of neutralizing by adding an acidic solution; a dialysis step; a grinding step; a final concentration adjustment step; a mixing step; and a sterilization step.

7. The method of claim 4, wherein the coupling agent comprises one or more of an activator and a coupling reagent.

8. A crosslinked hyaluronic acid, comprising hyaluronic acid, ornithine, and STMP.

9. The crosslinked hyaluronic acid of claim 8, wherein the crosslinked hyaluronic acid is capable of controlling the concentrations of hyaluronic acid, ornithine, and STMP according to desired storage modulus.

10. A composition for tissue repair, comprising the crosslinked hyaluronic acid of claim 9.

11. A filler composition, comprising the crosslinked hyaluronic acid of claim 9.

12. A composition for *in vivo* administration, comprising the crosslinked hyaluronic acid of claim 9.
